# EUROPEAN PATENT SPECIFICATION

(11) **EP 1 092 781 B1**
(45) Date of publication and mention of the grant of the patent: **01.03.2006**
(21) Application number: 99307617.3
(22) Date of filing: 28.09.1999
(51) Int. Cl.: C12P 7/18, C12N 1/16, C12R 1/72

(54) **Fermentation process for preparing erythritol by a high salt tolerant mutant of candida sp.**
Fermentations-Verfahren zur Herstellung von Erythritol mittels eines Mutanten von Candida sp. mit hoher Salztoleranz
Procédé de production d'Erythritol par fermentation en utilisant un mutant de candida sp. ayant une tolérance élévée au sel

(43) Date of publication of application: 18.04.2001
(73) Proprietor: Bolak Co., Ltd., Kyunggi-Do (KR)
(72) Inventor: Seo, Jin Ho Imkwang Apt 311-601, Sungnam-Shi (KR); Ryu,Yeon Woo Hyundai Apt 31-1005, Kangnam-Ku, Seoul (KR); Jung,Soo Ryun Asia Sunsoochon Apt 1-901, Songpa-Ku,Seoul (KR); Kim, Sang Yong Joogong Apt 1214-404, Kwangmyung-Shi,Kyunggi-Do (KR)
(74) Representative: Harding, Charles Thomas

(56) References cited:
- CHEMICAL ABSTRACTS, vol. 81, no. 5, 5 August 1974 (1974-08-05) Columbus, Ohio, US; abstract no. 22908, HATTORI, KIYOJI ET AL: "Production of erythritol by n-alkane-grown yeasts" XP002133023 & AGR. BIOL. CHEM. (1974), 38(3), 581-6 ,
- DATABASE WPI Section Ch, Week 199902 Derwent Publications Ltd., London, GB; Class D16, AN 1999-022095 XP002133025 & KR 98 002 246 A (CHEIL FOODS & CHEM INC) , 30 March 1998 (1998-03-30)
- CHEMICAL ABSTRACTS, vol. 132, Columbus, Ohio, US; abstract no. 34838, YANG, SUNG-WOOK ET AL: "Production of erythritol from glucose by an osmophilic mutant of Candida magnoliae" XP002133024 & BIOTECHNOL. LETT. (1999), 21(10), 887-890 ,

## Description

### BACKGROUND OF THE INVENTION

### 1. Field of the Invention

The present invention relates to a fermentation process for preparing erythritol to have a high productivity with a salt tolerant mutant of *Candida* sp., [*Candida magnoliae* SR101 : KCCM-10160] more specifically, for preparing erythritol under optimal fermentation conditions for maximal erythritol production by optimizing the environmental conditions of culture such as medium component, pH, temperature, aeration rate and agitation speed.

### 2. Description of Prior Art

Erythritol, a four carbon sugar alcohol, is a naturally occurring substance and is widely distributed in nature. Like most of the other polyols, it is a metabolite or storage compound for seaweeds and mushrooms. Fruits like melons, grapes and pears also contain erythritol. As it is often produced by bacteria, fungi, and yeasts, erythritol also occurs frequently in fermented food systems like wines or beers, and processed vegetables such as soy sauce or the oriental miso bean paste.

Erythritol is a moderately sweet bulking agent with 60~70 percent of the sweetness of sucrose in a ten percent solution. Its high positive enthalpy of solution provides the crystalline material with a strong cooling effect. As it has a taste which is very close to sucrose without bitter aftertaste, it is ideal to improve the taste of a combination with intense sweeteners like aspartame.

Being a small molecule, erythritol has strong colligative properties, i.e. a strong freezing point depression and boiling point elevation effect as well as a high osmotic pressure. In combination with its low hygroscopicity and viscosity in solution, it is very useful to reduce and control the water activity of foodstuffs.

Erythritol production from its natural sources such as fruits and vegetables is impractical due to the relative small amounts. Erythritol can be chemically produced by reduction of meso-tartarate, oxidation and reduction of 4,6-*o*-ethylidene-D-glucose, hydrolysis of dealdehyde starch, or addition of hydrogen. Since erythritol production by the chemical methods has been found to be expensive, it is worthwhile to explore an alternative method for the effective production of erythritol using microorganisms.

Erythritol can be produced by microbial methods with the osmophilic yeasts, especially species of the genus *Torulopsis,* such as *T. magnoliae, T. veratilis,* and *T. candida; Endomycopsis chodati; Hansenula supelliculsa; Pichia miso; Monilliella tomentosa var. pollinis; Trigonopsis variabilis; Trichosporonoides; Candida zeylanoides;* and *Aureobasidium*. Some bacteria such as *Leuconostoc oenos* can also produce erythritol. *Monilliella tomentosa var. pollinis* produced erythritol on a medium containing 35.7% glucose with 45.6% yield. Erythritol production using this strain did not apply to industrial scale due to by-products such as glycerol and ribitol. Industrial production of erythritol has been performed by a mutant of *Aureobasidium*. The mutant was isolated and developed by cooperative study of Nikken Chemical and National Research Institute of Japan. The mutant produced erythritol with 47.6% yield on a medium containing 22.5% glucose and 2 g/L-h volumetric productivity. However, the culture with this fugus had more difficultly than that with yeast. A method for producing erythritol using Candida zeylanoides is described in Hatton & Suzuki (1974) Agri Biol. Chem 38, 581-586.

Therefore, in this invention, a wild yeast strain of *Candida* sp. isolated from nature was mutated with EMS (Ethyl-methanol sulfonate) treatment. One of mutants has superior properties to the wild strain in erythritol yield from glucose, volumetric productivity, and salt tolerance. By using the mutant of *Candida* sp., the optimization of the environmental conditions of culture was performed for maximal erythritol production.

### SUMMARY OF THE INVENTION

The object of the present invention is to provide novel mutants cells of *Candida magnoliae* SR101, which were deposited to Korean Culture Center of Microorganism with accession number KCCM-10160 on May 21, 1999 under Budapest treaty, for preparing erythritol with high productivity.

The other object of the present invention is to provide the optimal fermentation process for maximum production of erythritol using mutant cells of *Candida magnoliae* SR101 deposited to Korean Culture Center of Microorganism with accession number KCCM-10160 comprising the steps of ;
i) fermenting monosaccharide or disaccharide medium with cells by controlling following fermentation conditions ;
   a) composition of medium for maximum production of erythritol consists of 10~50(w/v)% of glucose, 0.2~2.0(w/v)% of yeast extract, 0.1~ 10(w/v)% of KH₂PO₄, 0.1~5.0(w/v)% of (NH₄)₂SO₄ and 0.01~ 1.0(w/v)% of MgSO₄·7H₂O.
   b) pH of culture medium is 6~8.
   c) temperature of cultivation is 26~ 30°C.
   d) aeration rate is 0.75~2.0 volume of air per volume of medium per minute.
   e) agitation speed is 300~1200 rpm.
ii) feeding solution containing KCl continuously or intermittently fed into the culture broth during erythritol production phase to be 2~10% of its concentration.
iii) removing cells from the fermentation medium ; and
iv) separating and recovering erythritol from the fermentation medium of step iii)

### DETAILED DESCRIPTION OF THE INVENTION

The present invention concerns a method of obtaining erythritol with a high yield and a high volumetric productivity using mutant cells of *Candida magnoliae* by optimizing culture conditions.

The mutant cells used for the present invention are isolated by following method.

*Candida* sp. was screened from a comb. A piece of comb was transferred into the medium containing 40% of glucose and 1.0% of yeast extract, and incubated at 30°C. The broth was diluted and incubated at 30°C on agar plate containing 20% of glucose, 1.0% of yeast extract and 2.0% of agar. After obtained colonies were incubated on fermentation medium, which consisted of 10% of glucose and 1.0% of yeast extract, the culture broth was centrifuged to remove cells, and the supernant was analayzed for erythritol determination. A high erythritol producing strain was selected for erythritol production. The strain was identified to *Candida magnoliae* by Microcheck Co.

This strain was incubated on growth medium containing 2.0% of glucose, 1.0% of yeast extract and 1.0% of peptone. After growth, the broth was spread on agar plate containing 10% of glucose, 0.8% of yeast extract, 0.3% of peptone and 2.0% of agar, and then obtained colony was transferrred on sporulation medium containing 0.1% of glucose, 1.0% of yeast extract and 2.0% of agar. The formed spore was harvested by autoclaved distilled water and was selected by adding 10 mM of 2-mercaptoethanol for 30 min and treating lyticase 0.5 mg/ml for 4 hours.

The selected spore was treated by EMS (ethylmethanol sulfonate), and was incubated on the medium containing 30% of glucose, 18% of KCl, 0.1% of yeast extract and 2.0% of agar. Single colony was selected as fast growing mutants for the selection of a high salt tolerant mutant. The selected colony was transferred on the fermentation medium to test erythritol producing activity in shake flask.

After incubating at 30°C and 240 rpm for 72 hours, a high erythritol producing mutant was selected. Finally, growing colony was isolated and obtained as mutant cells, and used as a producing strain in this invention. These mutant cells were deposited to Korean Culture Center of Microorganism with accession number KCCM-10160 under Budapest treaty.

The following is fermentation method for producing erythritol using mutants cells.

### Seed Culture

The cells of *Candida magnoline* (KCCM-10160) are cultivated in a 250-mL flask containing 40-60 mL of growth medium (2.0% of glucose, 1.0% of peptone, 1.0% of yeast extract) at 30°C and 240 rpm for 48 h and this seed culture was transferred to a 250-ml flask or a 5-L fermentor for producing erythritol in main culture.

### Main Culture

Flask experiments with fermentation medium were performed at 26~30°C and 300~1200 rpm in 60~100 hours. The fermentation medium consisted of 10~50% of glucose as carbon source and 0.2~2.0% of yeast extract, 0.1~ 5.0% of (NH₄)₂SO₄, 0.1~10% of KH₂PO₄ and 0.01~1.0% of MgSO₄·7H₂O were used to be inorganic sources. For the experimental purpose, glucose concentration was adjusted. Batch and fed-batch culture in the fermentor were performed at 26~30°C and initial pH 7. Aeration rate was in the range of 0.75~2.0 vvm. Agitation speed was 300~1200 rpm. Fed-batch culture was performed with 5~10% of glucose by adding continuously or intermittently 10~40% of glucose.

The fermentation process is preferably by fed-batch process. After glucose was completely consumed in the medium, the amount of erythritol is measured by high performance liquid chromatography equipped with Carbohydrate Analysis column. Dry cell weight is estimated by using a calibration curve made from relationship between optical density at 600 nm and dry cell weight. Glucose is measured by dinitrosalicylic acid method.

The measured yield of erythritol is 35~55% of glucose consumption and volumetric productivity is 0.7 g/L-h.

Finally the fermentation medium is centrifuged for removing cells and other residue, and the supernatant is filtered and dialyzed for obtaining erythritol.

The present invention can be explained more specifically by following examples.

### (EXAMPLE I) Isolation of mutant cells

*Candia* sp. was screened from a comb. A piece of comb was transferred into the medium containing 40% of glucose and 1.0% of yeast extract and incubated at 30°C. The broth was diluted and incubated at 30°C on agar plate containing 20% of glucose, 1.0% of yeast extract and 2.0% of agar. After obtained colonies were incubated on fermentation medium, which consisted of 10% of glucose and 1.0% of yeast extract, the culture broth was centrifuged to remove cells and the supernant was analyzed for erythritol determination. A high erythritol producing strain was selected for erythritol production. The strain was identified to *Candida magnoliae* by Microcheck Co.

This strain was incubated at 30°C for 48 hours on growth medium containing 2.0% of glucose, 1.0% of yeast extract and 1.0% of peptone. After growth, the broth was spread on agar plate containing 10% of glucose, 0.8% of yeast extract, 0.3% of peptone and 2.0% of agar at 30°C for 36 hours, and then obtained colony was transferrred on sporulation medium containing 0.1% of glucose, 1.0% of yeast extract and 2.0% of agar, and spores were formed at 4°C after 4 days. The formed spore was harvested by autoclaved distilled water and was selected by adding 10 mM of 2-mercaptoethanol for 30 min and treating lyticase 0.5 mg/ml (100,000 units) for 4 hours. The selected spore was treated for 30 min by EMS. The reaction was terminated by adding 5% of thiosulfate. The spore was incubated on the medium containing 30% of glucose, 18% of KCl, 0.1% of yeast extract and 2.0% of agar for the selection of a high salt tolerant mutant. Single colony was selected as fast growing mutants. The selected colonies were transferred on the fermentation medium of 50 mL to test erythritol producing activity in 250 mL-shake flask. After incubating at 30°C and 240 rpm in 72 hours, a high erythritol producing mutant was selected. Finally, growing colony was isolated and obtained as mutant cells and used as a producing strain in this invention. These mutant cells were deposited to Korean Culture Center of Microorganism with accession number KCCM-10160.

### (EXAMPLE II) Erythritol production by the fermentation of mutant cells

The mutant cells of *Candida magnoliae* (KCCM-10160) are cultivated in a 250-mL flask containing 50 mL of growth medium (2.0% of glucose, 1.0% of peptone, 1.0% of yeast extract) at 30°C and 240 rpm for 48 hours and this seed culture was transferred to a 250-ml flask for producing erythritol. Flask experiments with fermentation medium were performed at 28°C, initial pH 7, and 240 rpm for 84 hours. The fermentation medium consisted of 25% of glucose as carbon source and 0.5% of yeast extract, 0.2% of (NH₄)₂SO₄, 0.5%, of KH₂PO₄ and 0.04% of MgSO₄·7H₂O.

After 84 hours fermentation, the amount of erythritol from 10% of glucose is measured by HPLC equipped with Carbohydrate Analysis column. The obtained erythritol is 25 g/L and volumetric productivity is 0.30 g/L-h.

### (COMPARATIVE EXAMPLE I)

### Erythritol production by the fermentation of wild type of cells

The wild type of cells of *Candida magnoliae* are cultivated in a 250-mL flask containing 50 mL of growth medium (2.0% of glucose, 1.0% of peptone, 1.0% of yeast extract) at 30°C and 240 rpm for 48 hours, and this seed culture was transferred to a 250-ml flask for producing erythritol. Flask experiments with fermentation medium were performed at 28°C, initial pH 7, and 240 rpm for 108 hours. The fermentation medium consisted of 25% of glucose as carbon source and 0.5% of yeast extract, 0.2% of (NH₄)₂SO₄, 0.5% of KH₂PO₄ and 0.04% of MgSO₄·7H₂O.

After 108 hours fermentation, the amount of erythritol from 10% of glucose is measured by HPLC equipped with Carbohydrate Analysis column. The obtained erythritol is 17 g/L and volumetric productivity is 0.16 g/L-h.

### (EXAMPLE III) The effect of initial pH for erythritol production

The mutant cells of *Candida magnoliae* (KCCM-10160) are cultivated in a 250-mL flask containing 50 mL of growth medium (20% of glucose, 1.0% of peptone, 1.0% of yeast extract) at 30°C and 240 rpm for 48 hours, and this seed culture was transferred to a 250-ml flask for producing erythritol. Flask experiments with fermentation medium were performed at 28°C and 240 rpm for 84 hours. The fermentation medium consisted of 25% of glucose as carbon source and 0.5% of yeast extract, 0.2% of (NH₄)₂SO₄, 0.5% of KH₂PO₄ and 0.04% of MgSO₄·7H₂O. Effect of pH on erythritol production was investigated.

After 84 hours fermentation, the amount of erythritol at initial pH of 5.0 is measured by HPLC equipped with Carbohydrate Analysis column. The obtained erythritol is 21.4 g/L and volumetric productivity is 0.25 g/L-h.

After 84 hours fermentation, the amount of erythritol at initial pH of 6.0 is measured by HPLC equipped with Carbohydrate Analysis column. The obtained erythritol is 23.6 g/L and volumetric productivity is 0.28 g/L-h.

After 84 hours fermentation, the amount of erythritol at initial pH of 7.0 is measured by HPLC equipped with Carbohydrate Analysis column. The obtained erythritol is 25.0 g/L and volumetric productivity is 0.30 g/L-h.

After 84 hours fermentation, the amount of erythritol at initial pH of 5.0 is measured by HPLC equipped with Carbohydrate Analysis column. The obtained erythritol is 18.6 g/L and volumetric productivity is 0.22 g/L-h.

### (EXAMPLE IV) The effect of temperature for erythritol production

The mutant cells of *Candida magnoliae* (KCCM-10160) are cultivated in a 250-mL flask containing 50 mL of growth medium (2.0% of glucose, 1.0% of peptone, 1.0% of yeast extract) at 30°C and 240 rpm for 48 hours, and this seed culture was transferred to a 250-ml flask for producing erythritol. Flask experiments with fermentation medium were performed at initial pH of 7.0 and 240 rpm for 84 hours. The fermentation medium consisted of 25% of glucose as carbon source and 0.5% of yeast extract, 0.2% of (NH₄)₂SO₄, 0.5% of KH₂PO₄ and 0.04% of MgSO₄·7H₂O. Effect of temperature on erythritol production was investigated.

After 84 hours fermentation, the amount of erythritol at 26°C is measured by HPLC equipped with Carbohydrate Analysis column. The obtained erythritol is 15.0 g/L and volumetric productivity is 0.18 g/L-h.

After 84 hours fermentation, the amount of erythritol at 28°C is measured by HPLC equipped with Carbohydrate Analysis column. The obtained erythritol is 25.0 g/L and volumetric productivity is 0.30 g/L-h.

After 84 hours fermentation, the amount of erythritol at 30°C is measured by HPLC equipped with Carbohydrate Analysis column. The obtained erythritol is 20.7 g/L and volumetric productivity is 0.25 g/L-h.

### (EXAMPLE V) The effect of the KCl concentration for erythritol production

The mutant cells of *Candida magnoliae* (KCCM-10160) are cultivated in a 250-mL flask containing 50 mL of growth medium (2.0% of glucose, 1.0% of peptone, 1.0% of yeast extract) at 30°C and 240 rpm for 48 hours, and this seed culture was transferred to a 250-ml flask for producing erythritol. Flask experiments with fermentation medium were performed at 28°C and 240 rpm for 84 hours. The fermentation medium consisted of 25% of glucose as carbon source and 0.5% of yeast extract, 0.2% of (NH₄)₂SO₄, 0.5% of KH₂PO₄ and 0.04% of MgSO₄·7H₂O. Effect of KCl concentration on erythritol production was investigated.

After 84 hours fermentation, the amount of erythritol at 0.0% KCl is measured by HPLC equipped with Carbohydrate Analysis column. The obtained erythritol is 25.0 g/L and volumetric productivity is 0.30 g/L-h.

After 84 hours fermentation, the amount of erythritol at 1.0% KCl is measured by HPLC equipped with Carbohydrate Analysis column. The obtained erythritol is 25.2 g/L and volumetric productivity is 0.30 g/L-h.

After 84 hours fermentation, the amount of erythritol at 3.0% KCl is measured by HPLC equipped with Carbohydrate Analysis column. The obtained erythritol is 23.3 g/L and volumetric productivity is 0.28 g/L-h.

After 84 hours fermentation, the amount of erythritol at 5.0% KCl is measured by HPLC equipped with Carbohydrate Analysis column. The obtained erythritol is 27.6 g/L and volumetric productivity is 0.33 g/L-h.

After 84 hours fermentation, the amount of erythritol at 6.0% KCl is measured by HPLC equipped with Carbohydrate Analysis column. The obtained erythritol is 26.5 g/L and volumetric productivity is 0.32 g/L-h.

### (COMPARATIVE EXAMPLE II)

### The effect of the KCl concentration for erythritol production using wild type of cells

The wild type of cells of *Candida magnoliae* are cultivated in a 250-mL flask containing 50 mL of growth medium (2.0% of glucose, 1.0% of peptone, 1.0% of yeast extract) at 30°C and 240 rpm for 48 hours, and this seed culture was transferred to a 250-ml flask for producing erythritol. Flask experiments with fermentation medium were performed at 28°C and 240 rpm for 108 hours. The fermentation medium consisted of 25% of glucose as carbon source and 0.5% of yeast extract, 0.2% of (NH₄)₂SO₄, 0.5% of KH₂PO₄ and 0.04% of MgSO₄·7H₂O. Effect of KCl concentration on erythritol production was investigated.

After 84 hours fermentation, the amount of erythritol at 0.0% KCl is measured by HPLC equipped with Carbohydrate Analysis column. The obtained erythritol is 17.0 g/L and volumetric productivity is 0.16 g/L-h.

After 108 hours fermentation, the amount of erythritol at 1.0% KCl is measured by HPLC equipped with Carbohydrate Analysis column. The obtained erythritol is 24.0 g/L and volumetric productivity is 0.22 g/L-h.

After 108 hours fermentation, the amount of erythritol at 2.0% KCl is measured by HPLC equipped with Carbohydrate Analysis column. The obtained erythritol is 23.7 g/L and volumetric productivity is 0.26 g/L-h.

After 108 hours fermentation, the amount of erythritol at 3.0% KCl is measured by HPLC equipped with Carbohydrate Analysis column. The obtained erythritol is 14.2 g/L and volumetric productivity is 0.13 g/L-h.

### (EXAMPLE VI) The erythritol production according to the change of aeration in the fermentor

The mutant cells of *Candida magnoliae* (KCCM-10160) are cultivated in a 250-mL flask containing 50 mL of growth medium (2.0% of glucose, 1.0% of peptone, 1.0% of yeast extract) at 30°C and 240 rpm for 48 hours, and this seed culture was transferred to a 5-L fermentor for producing erythritol. Fermentor experiments with fermentation medium were performed at 28°C, initial pH of 7.0, and 500 rpm. The fermentation medium consisted of 25% of glucose as carbon source and 0.5% of yeast extract, 0.2% of (NH₄)₂SO₄, 0.5% of KH₂PO₄, 5.0% of KCl, and 0.04% of MgSO₄·7H₂O. Effect of aeration rate on erythritol production was investigated.

After 205 hours fermentation, the amount of erythritol at 0.75 vvm is measured by HPLC equipped with Carbohydrate Analysis column. The obtained erythritol is 131.2 g/L and volumetric productivity is 0.64 g/L-h.

After 205 hours fermentation, the amount of erythritol at 1.00 vvm is measured by HPLC equipped with Carbohydrate Analysis column. The obtained erythritol is 143.3 g/L and volumetric productivity is 0.70 g/L-h.

After 205 hours fermentation, the amount of erythritol at 1.50 vvm is measured by HPLC equipped with Carbohydrate Analysis column. The obtained erythritol is 125.0 g/L and volumetric productivity is 0.61 g/L-h.

### (EXAMPLE VII) The erythritol production according to the change of glucose concentration in the fermentor

The mutant cells of *Candida magnoliae* (KCCM-10160) are cultivated in a 250-mL flask containing 50 mL of growth medium (2.0% of glucose, 1.0% of peptone, 1.0% of yeast extract) at 30°C and 240 rpm for 48 hours, and this seed culture was transferred to a 5-L fermentor for producing erythritol. Fermentor experiments with fermentation medium were performed at 28°C, initial pH of 7.0, 1.0 vvm, and 500 rpm for 84 hours. The fermentation medium consisted of glucose as carbon source and 0.5% of yeast extract, 0.2% of (NH₄)₂SO₄, 0.5% of KH₂PO₄, 5.0% of KCl and 0.04% of MgSO₄·7H₂O. Effect of glucose concentration on erythritol production was investigated.

After 63 hours fermentation, the amount of erythritol at 10% of glucose is measured by HPLC equipped with Carbohydrate Analysis column. The obtained erythritol is 29.1 g/L and volumetric productivity is 0.46 g/L-h.

After 120 hours fermentation, the amount of erythritol at 15% of glucose is measured by HPLC equipped with Carbohydrate Analysis column. The obtained erythritol is 64.9 g/L and volumetric productivity is 0.54 g/L-h.

After 160 hours fermentation, the amount of erythritol at 20% of glucose is measured by HPLC equipped with Carbohydrate Analysis column. The obtained erythritol is 87.2 g/L and volumetric productivity is 0.55 g/L-h.

After 205 hours fermentation, the amount of erythritol at 25% of glucose is measured by HPLC equipped with Carbohydrate Analysis column. The obtained erythritol is 143 g/L and volumetric productivity is 0.70 g/L-h.

After 205 hours fermentation, the amount of erythritol at 30% of glucose is measured by HPLC equipped with Carbohydrate Analysis column. The obtained erythritol is 117 g/L and volumetric productivity is 0.57 g/L-h.

### (EXAMPLE VIII) The erythritol production according to the change of sucrose concentration in the fermentor

The mutant cells of *Candida magnoliae* (KCCM-10160) are cultivated in a 250-mL flask containing 50 mL of growth medium (2.0% of glucose, 1.0% of peptone, 1.0% of yeast extract) at 30°C and 240 rpm for 48 hours, and this seed culture was transferred to a 5-L fermentor for producing erythritol. Fermentor experiments with fermentation medium were performed at 28°C, initial pH of 7.0, 1.0 vvm, and 500 rpm for 84 hours. The fermentation medium consisted of sucrose as carbon source and 0.5% of yeast extract, 0.2 % of (NH₄)₂SO₄, 0.5% of KH₂PO₄, 5.0% of KCl, and 0.04% of MgSO₄·7H₂O. Effect of sucrose concentration on erythritol production was investigated.

After 65 hours fermentation, the amount of erythritol at 10% of sucrose is measured by HPLC equipped with Carbohydrate Analysis column. The obtained erythritol is 26.1 g/L and volumetric productivity is 0.40 g/L-h.

After 154 hours fermentation, the amount of erythritol at 20% of sucrose is measured by HPLC equipped with Carbohydrate Analysis column. The obtained erythritol is 78.3 g/L and volumetric productivity is 0.51 g/L-h.

After 175 hours fermentation, the amount of erythritol at 20% of sucrose is measured by HPLC equipped with Carbohydrate Analysis column. The obtained erythritol is 105 g/L and volumetric productivity is 0.60 g/L-h.

After 250 hours fermentation, the amount of erythritol at 40% sucrose is measured by HPLC equipped with Carbohydrate Analysis column. The obtained erythritol is 126 g/L and volumetric productivity is 0.50 g/L-h.

*Candida magnoline* KCCM-10160 in the present can produce erythritol from glucose or sucrose with a high productivity and a high yield, make little foam, and does not produce by-products such as glycerol, resulting in easy recovery. These advantages have considerable importance for the possible commercial manufacture of erythritol.

## Claims

1. A fermentation process for production of erythritol using mutant cells of *Candida magnoliae* SR101 deposited to Korean Culture Center of Microorganism with accession number KCCM-10160 comprising the steps of ;
i) fermenting monosaccharide or disaccharide medium with cells by controlling following fermentation conditions ;
a) composition of medium for maximum production of erythritol consists of 10~50(w/v)% of glucose, 0.2~2.0(w/v)% of yeast extract, 0.1-10(w/v) % of KH₂PO₄, 0.1~5.0(w/v)% of (NH₄)₂SO₄ and 0.01~ 1.0(w/v)% of MgSO₄·7H₂O.
b) pH of culture medium is 6-8.
c) temperature of cultivation is 26~ 30°C.
d) aeration rate is 0.75~2.0 volume of air per volume of medium per minute.
e) agitation speed is 300~1200 rpm.
ii) feeding solution containing KCl continuously or intermittently fed into the culture broth during erythritol production phase to be 2~10% of its concentration.
iii) removing cells from the fermentation medium ; and
iv) separating and recovering erythritol from the fermentation medium of step iii)

2. The novel mutant cells of *Candida magnoliae* SR101 (KCCM-10160).

## Patentansprüche

1. Fermentationsverfahren für die Herstellung von Erythritol unter Verwendung mutanter Zellen von *Candida magnoliae* SR101, die beim Korean Culture Center of Microorganism unter der Hinterlegungsnummer KCCM-10160 hinterlegt wurden, welches die folgenden Stufen umfaßt:
i) Fermentieren von Monosaccharid- oder Disaccharidmedium mit Zellen unter Steuern der folgenden Fermentationsbedingungen:
a) das Medium für die Herstellung maximaler Mengen an Erythritol setzt sich aus 10-50 (w/v)% Glucose, 0,2-2,0 (w/v)% Hefeextrakt, 0,1-10 (w/v)% KH₂PO₄, 0,1-5,0 (w/v)% (NH₄)₂SO₄ und 0,01-1,0 (w/v)% MgSO₄ · 7 H₂O zusammen,
b) der pH-Wert des Kulturmediums beträgt 6-8,
c) die Kultivierungstemperatur beträgt 26-30°C,
d) die Belüftungsrate beträgt 0.75-2,0 Volumen Luft pro Volumen Medium pro Minute,
e) die Rührgeschwindigkeit beträgt 300-1200 U.p.M.,
ii) Zuführen einer Lösung, die KCl enthält, die kontinuierlich oder unterbrochen der Kulturbrühe während der Phase der Erythritolherstellung zugeführt wird, so daß diese 2-10% von dessen Konzentration enthält,
iii) Entfernen von Zellen aus dem Fermentationsmedium und
iv) Trennen und Gewinnen von Erythritol aus dem Fermentationsmedium aus Stufe iii).

2. Neuartige mutante Zellen von *Candida magnoliae* SR101 (KCCM-10160).

## Revendications

1. Procédé de fermentation pour la production d'érythritol en utilisant des cellules mutantes de *Candida magnoliae* SR101 déposées dans le Korean Culture Center of Microorganism sous le numéro de dépôt KCCM-10160, comprenant les étapes consistant :
i) à faire fermenter un milieu monosaccharidique ou disaccharidique avec les cellules en ajustant les conditions de fermentation suivantes :
a) la composition du milieu pour la production maximale d'érythritol consiste en 10 à 50 % (en poids/volume) de glucose, 0,2 à 2,0 % (en poids/volume) d'extrait de levure, 0,1 à 10 % (en poids/volume) de KH₂PO₄, 0,1 à 5,0 % (en poids/volume) de (NH₄)₂SO₄ et 0,01 à 1,0 % (en poids/volume) de MgSO₄.7H₂O.
b) le pH du milieu de culture est compris dans la plage de 6 à 8,
c) la température de culture est comprise dans l'intervalle de 26 à 30°C,
d) la vitesse d'aération est comprise dans l'intervalle de 0,75 à 2,0 volume d'air par volume de milieu par minute,
e) la vitesse d'agitation est comprise dans l'intervalle de 300 à 1200 tr/min ;
ii) à amener une solution contenant du KCl de manière continue ou intermittente dans le bouillon de culture au cours de la phase de production d'érythritol de manière à parvenir à une valeur de 2 à 10 % de sa concentration.
iii) à séparer les cellules du milieu de fermentation ; et
iv) à séparer et à recueillir l'érythritol à partir du milieu de fermentation de l'étape iii).

2. Cellules mutantes nouvelles de *Candida magnoliae* SR101 (KCCM-10160).
